(19) **Europäisches Patentamt**
**European Patent Office**
**Office européen des brevets**

(11) Publication number: **0 242 988 B1**

## (12) EUROPEAN PATENT SPECIFICATION

(45) Date of publication of patent specification:
**27.11.91 Bulletin 91/48**

(51) Int. Cl.⁵: **A61F 9/02, A41D 13/00**

(21) Application number: **87302399.8**

(22) Date of filing: **19.03.87**

(54) **Visor-type mask, especially for dentists.**

(30) Priority: **21.03.86 US 842150**

(43) Date of publication of application:
**28.10.87 Bulletin 87/44**

(45) Publication of the grant of the patent:
**27.11.91 Bulletin 91/48**

(84) Designated Contracting States:
**AT BE CH DE FR GB GR IT LI NL SE**

(56) References cited:
**CH-A- 317 863**
**DE-C- 519 567**
**FR-A- 480 364**

(56) References cited:
**FR-A- 1 477 618**
**FR-A- 1 527 271**
**US-A- 1 911 817**
**US-A- 2 774 970**
**US-A- 2 798 222**

(73) Proprietor: **Landis, Timothy J.**
**4532 Las Encinitas**
**Fair Oaks California 95628 (US)**

(72) Inventor: **Landis, Timothy J.**
**4532 Las Encinitas**
**Fair Oaks California 95628 (US)**

(74) Representative: **Bizley, Richard Edward et al**
**BOULT, WADE & TENNANT 27 Furnival Street**
**London EC4A 1PQ (GB)**

## Description

This invention relates to a face mask for use, for example, by dentists, surgeons, and others who may be contaminated with germs and viruses of their patients and customers.

Surgical masks of gauze and paper have been used by surgeons and sometimes by dentists to prevent intercontamination of the doctor and patient. However, wearing such masks is hot and uncomfortable, and frequently, frightening to patients. Putting the masks on and removing them are time consuming and often difficult. Breath condenses within the mask and the latter becomes saturated with moisture and thereby fails to be an effective barrier to viruses and bacteria.

Surgical masks cause the wearer to re-inhale exhaled breath causing the $CO_2$ content of the blood to rise. The result of this may be increased heart and respiration rates and higher body temperatures and perspiration.

Safety face shields with vents, but of different construction are shown in US-A-3,152,588; 3,298,031; 3,678,929; and 4,250,477.

Detachable masks are shown in GB-A-503,750 and US-A-1,279,884; 2,818,829; and 3,346,875. Mere face shields are shown in US-A-2,978,709 for example.

Additionally, shields attached to eyeglass-type frames are shown in DE-A-688,227; US-A-1,582,164 and 2,774,970.

FR-A-480364 discloses a face mask comprising a visor, first means supporting the visor which, in case of the mask, projects forwardly from the head of the wearer above the eye, and a shield which is transparent over at least a substantial portion of its area and extends down from the visor to below the mouth and around the face to protect the eyes, nose and mouth of the wearer.

This known mask consists of a light metal frame on which transparent materials (such as mica) are fitted. It is intended to be secured around the face to protect the wearer against wind and dust.

The present invention is characterised in that the shield is supported solely by the visor and in that second means detachably secures the shield to the visor, the first means comprising a resilient band having a centre portion to extend around the front of the head and discrete flexible ends terminating spaced from the back of head, and the second means comprising first fastening means on the visor and co-operating second fastening means on the shield.

The shield protects the eyes, nose and mouth of the wearer, but does not interfere with normal breathing. Coated on or otherwise applied to the shield may be a filter to protect the eyes from ultraviolet, blue or other harmful radiation from instruments which are presently used by dentists in curing tooth-filling plastic.

The visor may have a vent opening closed by a cover which may be raised and lowered to control ventilation.

The shield may be curved and downwardly tapered to protect the eyes, nose and mouth of the wearer from contamination. Between uses the shield may be cleaned or, if cost considerations are not too great, may be discarded to prevent cross-contamination of patients.

The shield protects the wearer from handpiece "splatter" and foreign objects such as filling and tooth structure and viruses. Burrs throw off saliva, tooth particles and the like. Similarly, high pressure water and air spread contaminants. These may enter the eye, nose and mouth of the dentist if not masked.

Since the shield is detachable, it may be removed between patients or whenever it becomes contaminated for cleaning or replacement.

There is provision in the mask for air circulation so that carbon dioxide build-up from re-breathing expelled air does not occur. Venting also prevents fogging the eye-glass lenses of the wearer if used. The vents are so located, however, that they do not defeat the anti-contamination features of the mask. Thus, the bottom of the shield is located forwardly of the mouth and nose, permitting air to flow up from below the face of the wearer into space under the shield. Further, as has been mentioned, a vent may be provided in the visor above the shield. Detritis from the mouth of the patient, however, cannot enter the vent because it is located out of a direct line of travel. If desired, a hinged or snap-in cover may be used to close off the vent in the visor.

In the accompanying drawings:

Figure 1 is a perspective view showing a mask in use;

Figure 2 is a side elevational view thereof;

Figure 3 is a front elevation;

Figure 4 is a bottom plan view;

Figure 5 is an enlarged fragmentary view showing the hinge of the closure for a vent;

Figure 6 is a fragmentary sectional view taken substantially along the line 6-6 of Figure 5;

Figures 7, 8, 9, 10 are views showing shields in flat condition prior to installation.

Figure 11 is a view similar to Figure 1 of a modification;

Figure 12 is an enlarged side elevation of the mask of Figure 11;

Figure 13 is a bottom plan thereof;

Figure 14 is a sectional view taken along line 14-14 of Figure 13;

Figure 15 is a fragmentary elevational view of a portion of Figure 13 developed in a plane; and

Figure 16 is an elevational view of a shield used with the modification of Figure 13 and also showing a modified filter.

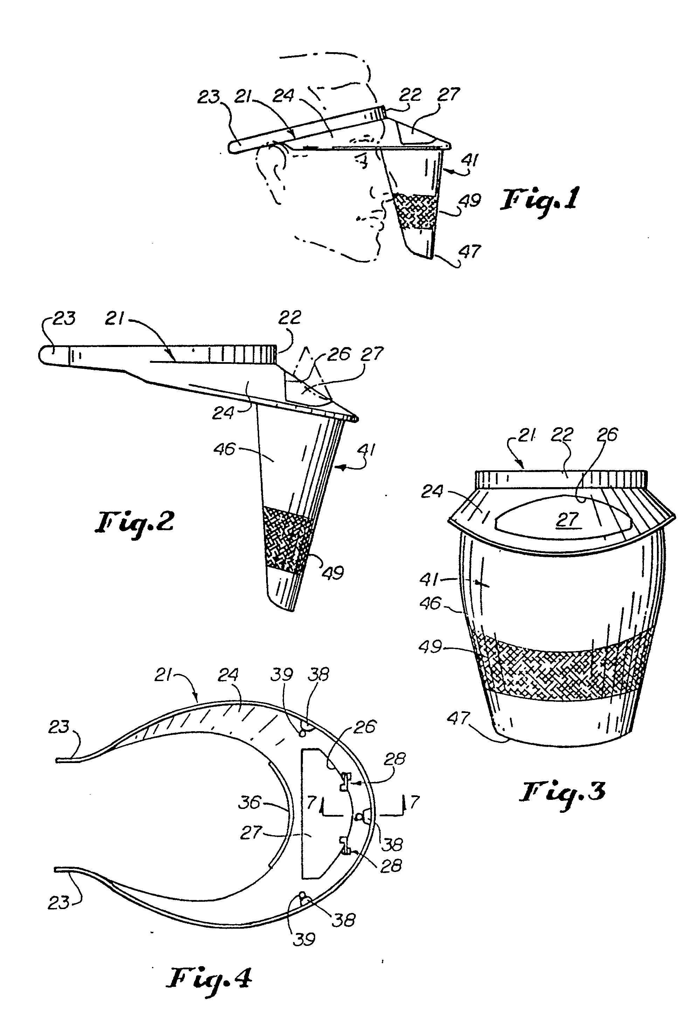

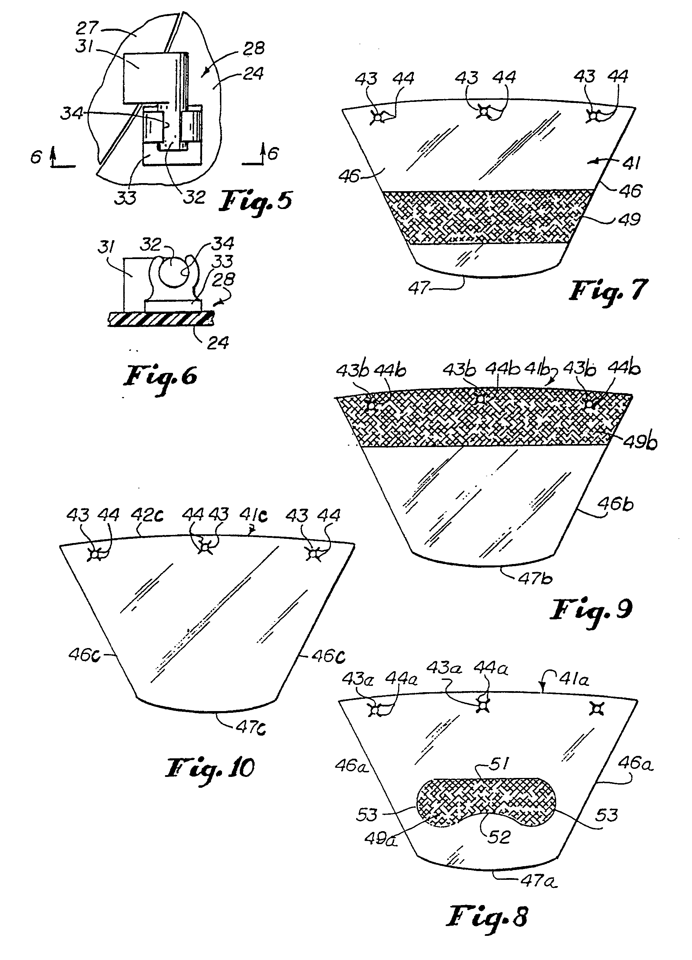

The face mask illustrated in Figures 1 to 10 comprises a band 21 of a material such as polyethylene which is resiliently flexible. The center portion 22 fits around the forehead of the wearer and extends around the sides of the head terminating in ends 23 on each side which grip the head sufficiently strongly to hold the device in place without creating discomfort. Integral with and extending forwardly from the band 21, is a visor 24 shaped in the manner of a conventional sun visor. In the center of the visor 24 is an opening 26, having a straight rear edge and a curved front edge, which may be closed off by a cover 27. The cover 27 is connected to the visor 24 adjacent to the front margin of the opening 26 by hinges 28. In the particular type of hinge illustrated herein (see Figures 5 and 6), there is a leaf 31 fixed to the forward edge of the cover 27 and having outwardly extending hinge pins 32. A leaf 33 is fixed to the visor 24 adjacent to the opening 26 on each side of the device and is formed with a socket 34 into which the pin 32 snaps. By the pin 32 oscillating within the socket 34, the cover 27 may be raised from the solid line position of Figure 2 to the dotted line position thereof. It will be understood that the details of construction of hinge 28 are subject to modification. In the raised position, ventilation is achieved. Nevertheless, the fact that the cover 27 in the open position slants upwardly-rearwardly prevents foreign objects from falling into the opening 26.

For comfort of the wearer, a resilient sponge-rubber pad 36 (Figure 4) is fixed to the inside of the band 21 to cushion contact of the latter with the skin of the wearer.

Fixed in a plurality of positions (here shown as three in number) on the lower edge of visor 24 are bosses 38 having studs 39 projecting therefrom.

A shield 41 (of which several varieties are illustrated and hereinafter described) is detachably secured to visor 24. As best shown in Figure 7, the shield 41 in the flat condition has a slightly arcuate upper edge 42 and formed immediately below the edge 42 are holes 43 spaced the same distance apart as the arcuate distances between the studs 39. To facilitate the studs 39 snapping into and out of the holes 43, radial slits 44 are formed in the shield 41 which provide prongs which engage under the studs 39. Thus, the shield 41 may be snapped onto the visor 24 or removed therefrom. Preferably the shield 41 is sufficiently inexpensive so that it may be discarded between uses but, if economy is a factor, the shield may be washed after being used for one patient and before being used for the next.

Although the shape of the shield 41 is subject to some modification, it covers the eyes, nose and mouth of the user so that in transverse horizontal section it is approximately semicircular. Thus, the sides 46 of the shield 41 converge, terminating in a bottom edge 47 which, as shown in Figure 1, is at a level about that of the chin of the user. Uncontaminated air enters behind the shield 41 and any breath expelled from the mouth is discharged from the atmosphere rather than being rebreathed. This is particularly facilitated when the cover 27 is in the open position.

For some uses, particularly in dentistry, a filter 49 of an orange plastic, which filters ultraviolet or high wavelength blue light, is disposed across the shield 41 at a level slightly below the nose. In normal use, the filter 49 is out of the line of sight through the shield 41. Dentists frequently use ultraviolet or blue light lamps (not shown) to cure plastic tooth-filling materials. By tilting the head upward and directing the eyes, the filter 49 may be interposed between the instrument emitting the ultraviolet light and the eyes of the wearer to protect them against damage. The filter 49 may be of other colours, depending upon conditions under which the mask is used. The outer surface of shield 41 in the region of the filter band 49 may be silvered to further filter undesired rays.

Figure 8 shows a filter 49a with a shape different from that of Figure 7 and having a straight upper edge 51 extending only partially across the width of the shield 41a and the underside edge 52 of the filter band 49a is curved upwardly. The sides 53 are rounded.

The mask of Figure 9 gives improved lateral vision and yet performs the necessary filtering function. In Figure 9, the filter band 49b is located at the extreme upper end of the shield 41b.

Figure 10 shows a shield 41c having no filter incorporated therein which may be suitable for users who do not need the protection of a filter by reason of the nature of their work.

In other respects, the shields of Figure 8, 9 and 10 resemble the preceding constructions and the same reference numerals followed by subscripts a, b and c respectively, designate corresponding parts.

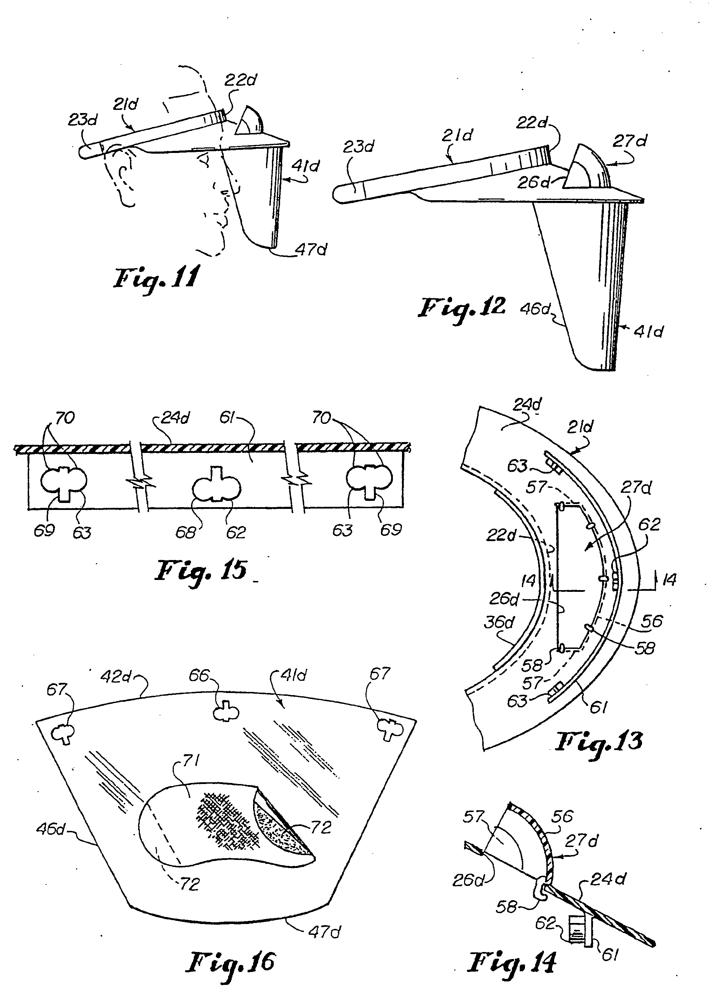

In Figures 11 to 16, the vent 26d in the visor 24d may be left open for ventilation purposes since the possibility of contaminating substances entering through the vent and contaminating the wearer of the mask is minimal. However, as a safety precaution, a modified cover 27d is illustrated. Such a cover has a forward side 56 which is curved into two planes and is provided with vertical ends 57 on each side. As shown in Figures 13 and 14, a plurality of hooks 58 are fixed to the bottom edge of side 56 and ends 57 which snap under the margins of the hole 26d. The cover 57 is sufficiently flexible by reason of its thin construction so that a person's fingers may be used to deform the cover 27d to snap the hooks 58 in place.

To reinforce the visor 24d and also to provide a means for attachment of the shield 41d thereto, an arcuate ledge 61 is formed projecting vertically down from the underside of the visor 24d. A center projection 62 and end projections 63 on each side extend from the rear face of the ledge 61. The projections 62 and 63 may be of various shapes. As shown in Figure 15, each projection 63 has a vertical leg 69 and on each side of the leg 69 are truncated round, flat projections 70. The center projection 62 is an inversion of the projections 63.

The shield 41d used with the projections 62 and 63 is formed with a center hole 66 and end holes 67 which are complementary to the projections 62 and 63 and are spaced apart a distance equal to the arcuate length between the projections 62 and 63.

Figure 16 shows a filter 71 which is detachable and re-usable. The shape of the filter 71 is subject to variation in that it may be similar to the filter 49 shown in Figure 1 or the filters 49a and 49b or any other shape. The filter 71 may be formed as a thin sheet of orange plastic material or other transparent or translucent material which filters out the ultraviolet or blue light heretofore mentioned. Along all or a portion of the right and left margins of the filter 71 are bands of pressure sensitive adhesive 72 which is used to attach the filter 71 in any position on the shield 41d, preferably on the rear face thereof to avoid contamination by spray or splatter.

The filter 71 may be applied in any position where it is most useful to the worker. It may be removed when the shield 41d is being cleaned and then replaced. Further, it will be understood that many users of the mask do not require a filter and, hence, a standard clear shield may be furnished and a supply of filters 71 provided which may be attached to perform the filtering function as desired.

In other respects the structure of Figures 11 to 16 resembles that of the preceding structures and the same reference numerals followed by the subscript d is used to designate corresponding parts.

## Claims

1. A face mask comprising a visor (24,24d), first means (21,21d) supporting the visor which, in use of the mask, projects forwardly from the head of the wearer above the eye level, and a shield (41,a-d) which is transparent over at least a substantial portion of its area and extends down from the visor to below the mouth and around the face to protect the eyes, nose and mouth of the wearer, characterised in that the shield is supported solely by the visor and in that second means (38,39,43,44; 62,63,66,67) detachably secures the shield (41) to the visor (24), the first means comprising a resilient band (21,21d) having a centre portion (22) to extend around the front of the head and discrete flexible ends (23) to engage the sides of the head of the wearer, the ends (23) terminating spaced from the back of the head, and the second means comprising first fastening means (38,39;62,63) on the visor and co-operating second fastening means (43,44; 66,67) on the shield.

2. A mask according to claim 1 in which the shield (41) is formed of a sheet of flexible transparent plastic having an upper edge and downward converging side edges.

3. A mask according to claim 1 or 2, in which the first fastening means comprises a plurality of studs (39) fixed to the underside of the visor (24) adjacent to the forward edge of the visor and the second fastening means comprises a plurality of holes (43) formed adjacent to the upper edge of the shield and spaced and shaped to resiliently receive the studs.

4. A mask according to claim 1 or 2, in which the first fastening means comprises a first central projection (62) and second and third projections (63) on opposite sides of the first projection, the shield (41d) having correspondingly positioned holes (66,67), each hole and respective projection corresponding in shape and size to each other and comprising a leg (69) with round portions (70) to each side.

5. A mask according to any preceding claim, in which the shield is provided with a filter (49) to protect the eyes from harmful rays.

6. A mask according to claim 5, in which the filter comprises a band of orange pigment across the shield having its upper edge below the level of the eyes.

## Patentansprüche

1. Gesichtsmaske mit einem Schirm (24, 24d), ersten Einrichtungen (21, 21d) zum Halten des Schirms, welcher bei Verwendung der Maske vom Kopf des Trägers über dem Augenniveau vorsteht, und mit einem Schutzschild (41, ad), der über wenigstens einem wesentlichen Abschnitt seiner Fläche transparent ist und sich von dem Schirm nach unten bis unterhalb des Mundes und um das Gesicht herum erstreckt, um die Augen, die Nase und den Mund des Trägers zu schützen, dadurch gekennzeichnet, daß der Schutzschild nur von dem Schirm gehalten ist und daß zweite Einrichtungen (38, 39, 43, 44; 62, 63, 66, 67) den Schutzschild (41) lösbar an dem Schirm (24) festlegen, wobei die ersten Einrichtungen ein elastisches Band (21, 21d) aufweisen, das einen Mittelabschnitt (22), der sich um die Vorderseite des Kopfes erstreckt, und getrennte flexible Enden (23) für den Eingriff an den Seiten des Kopfs des Trägers hat, die Enden (23) im Abstand von der Rückseite des Kopfes enden und die zweiten Einrichtungen erste Befestigungseinrichtungen (38, 39; 62, 63) an dem Schirm und zusammenwirkende zweite Befestigungseinrichtungen (43, 44; 66, 67) an dem Schild aufweisen.

2. Maske nach Anspruch 1, bei welcher der Schild (41) aus einer Platte eines flexiblen transparenten Kunststoffs hergestellt ist und einen oberen Rand und nach unten konvergierende Seitenränder aufweist.

3. Maske nach Anspruch 1 oder 2, bei welcher die ersten Befestigungseinrichtungen eine Vielzahl von Zapfen (39) aufweisen, die an der Unterseite des Schirms (24) angrenzend an den vorderen Rand des Schirms befestigt sind, und die zweiten Befestigungseinrichtungen eine Vielzahl von Löchern (43) aufweisen, die angrenzend an den oberen Rand des Schildes ausgebildet und in einem solchen Abstand angeordnet und so geformt sind, daß sie die Zapfen elastisch aufnehmen.

4. Maske nach Anspruch 1 oder 2, bei welcher die ersten Befestigungseinrichtungen einen ersten zentralen Vorsprung (62) und einen zweiten und dritten Vorsprung (63) auf gegenüberliegenden Seiten des ersten Vorsprungs aufweisen, der Schild (41d) entsprechend positionierte Löcher (66, 67) aufweist und jedes Loch und der jeweilige Vorsprung in Form und Größe einander entsprechen und einen Schenkel (69) mit runden Abschnitten (70) auf jeder Seite aufweisen.

5. Maske nach einem vorhergehenden Anspruch, bei welcher der Schild mit einem Filter (49) zum Schutz der Augen vor schädlichen Strahlen versehen ist.

6. Maske nach Anspruch 5, bei welcher der Filter ein Band aus orangenem Pigment quer über den Schirm aufweist, dessen oberer Rand unter dem Niveau der Augen liegt.

visière (24) à proximité immédiate du bord avant de la visière, et le second moyen de fixation comprend plusieurs trous (43) formés à proximité immédiate du bord supérieur de l'écran et espacés et façonnés de façon à recevoir élastiquement les ergots.

4. Masque selon la revendication 1 ou 2, dans lequel le premier moyen de fixation comporte une première saillie centrale (62) et des deuxième et troisième saillies (63) sur des côtés opposés de la première saillie, l'écran (41d) ayant des trous (66, 67) occupant des positions correspondantes, chaque trou et chaque saillie respective correspondant en forme et en dimension l'un à l'autre et comprenant une branche (69) avec des parties arrondies (70) sur chaque côté.

5. Masque selon l'une quelconque des revendications précédentes, dans lequel l'écran est pourvu d'un filtre (49) destiné à protéger les yeux de rayons nocifs.

6. Masque selon la revendication 5, dans lequel le filtre comprend une bande d'un pigment orange traversant l'écran, ayant son bord supérieur au-dessous du niveau des yeux.

**Revendications**

1. Masque facial comportant une visière (24, 24d), un premier moyen (21, 21d) supportant la visière qui, lors de l'utilisation du masque, fait saillie vers l'avant de la tête du porteur au-dessus du niveau des yeux, et un écran (41, ad) qui est transparent sur au moins une partie importante de son étendue et qui s'étend vers le bas depuis la visière jusqu'au-dessous de la bouche et autour de la face pour protéger les yeux, le nez et la bouche du porteur, caractérisé en ce que l'écran est supporté uniquement par la visière et en ce qu'un second moyen (38, 39, 43, 44 ; 62, 63, 66, 67) fixe de façon détachable l'écran (41) à la visière (24), le premier moyen comportant un bandeau élastique (21, 21d) ayant une partie centrale (22) destinée à s'étendre le long du devant de la tête et des extrémités flexibles et distinctes (23) destinées à porter contre les côtés de la tête du porteur, les extrémités (23) se terminant à distance de l'arrière de la tête, et le second moyen comportant un premier moyen de fixation (38, 39 ; 62, 63) sur la visière et un second moyen coopérant de fixation (43, 44 ; 66, 67) sur l'écran.

2. Masque selon la revendication 1, dans lequel l'écran (41) est formé d'une feuille de matière plastique flexible et transparente ayant un bord supérieur et des bords latéraux convergeant vers le bas.

3. Masque selon la revendication 1 ou 2, dans lequel le premier moyen de fixation comporte plusieurs ergots (39) fixés à la face inférieure de la

23 21 24 22 27 41 49 47

*Fig.1*

23 21 22 26 27 24 46 41 49

*Fig.2*

21 22 26 24 27 41 46 49 47

*Fig.3*

21 24 39 38 23 26 28 36 7 7 27 28 38 23 38 28 39

*Fig.4*

Fig.5

Fig.6

Fig. 7

Fig. 9

Fig. 10

Fig.8

Fig. 11

Fig. 12

Fig. 15

Fig. 13

Fig. 16

Fig. 14